# FASCICULE DE BREVET EUROPEEN

(11) **EP 3 615 014 B1**
(45) Date de publication et mention de la délivrance du brevet: **06.10.2021**
(21) Numéro de dépôt: 18717085.7
(22) Date de dépôt: 18.04.2018
(51) Int. Cl.: A61K 31/135, A61K 9/00, A61K 9/10, A61P 25/02

(54) **COMPOSITION PHARMACEUTIQUE TOPIQUE COMPRENANT AU MOINS DE L'AMITRIPTYLINE POUR LE TRAITEMENT DE DOULEURS NEUROPATHIQUES PÉRIPHÉRIQUES**
TOPISCHE PHARMAZEUTISCHE ZUSAMMENSETZUNG MIT MINDESTENS AMITRIPTYLIN ZUR BEHANDLUNG VON PERIPHEREM NEUROPATHISCHEM SCHMERZ
TOPICAL PHARMACEUTICAL COMPOSITION COMPRISING AT LEAST AMITRIPTYLINE, FOR THE TREATMENT OF PERIPHERAL NEUROPATHIC PAIN

(30) Priorité: 25.04.2017 FR 1753577
(43) Date de publication de la demande: 04.03.2020
(73) Titulaire: Algotherapeutix, 92150 Suresnes (FR)
(72) Inventeur: GRÉCO, Céline, 75012 Paris (FR)
(74) Mandataire: Casalonga
(86) Numéro de dépôt international: PCT/EP2018/059948
(87) Numéro de publication internationale: WO 2018/197307

(56) Documents cités:
- US-A1- 2002 028 789
- US-A1- 2004 101 582
- GEWANDTER JENNIFER S ET AL: "A phase III randomized, placebo-controlled study of topical amitriptyline and ketamine for chemotherapy-induced peripheral neuropathy (CIPN): a University of Rochester CCOP study of 462 cancer survivors", SUPPORTIVE CARE IN CANCER, vol. 22, no. 7, 16 février 2014 (2014-02-16), pages 1807-1814, XP035316150, SPRINGER VERLAG, BERLIN, DE ISSN: 0941-4355, DOI: 10.1007/S00520-014-2158-7 [extrait le 2014-02-16]
- D.L.BARTON ET AL: "A double blind, placebo controlled trial of a topical treatment for chemotherapy induced peripheral neuropathy: NCCTG trial N06CA", SUPPORTIVE CARE IN CANCER, vol. 19, no. 6, juin 2011 (2011-06), pages 833-841, XP002782791,
- DAVID J. KOPSKY ET AL: "High Doses of Topical Amitriptyline in Neuropathic Pain: Two Cases and Literature Review : Topical Amitriptyline in Neuropathic Pain", PAIN PRACTICE, vol. 12, no. 2, 16 juin 2011 (2011-06-16), pages 148-153, XP055434409, US ISSN: 1530-7085, DOI: 10.1111/j.1533-2500.2011.00477.x

## Description

La présente invention concerne une composition pharmaceutique comprenant de 10 à 30 % en poids, par rapport au poids total de la composition, d'amitriptyline ou de l'un de ses sels pharmaceutiquement acceptable, pour son utilisation dans le traitement de douleurs neuropathiques périphériques post-chimiothérapie par voie topique.

La douleur neuropathique périphérique est causée par l'endommagement des structures nerveuses telles que les terminaisons nerveuses périphériques ou les nocicepteurs qui deviennent extrêmement sensibles à la stimulation et qui peuvent générer des impulsions en l'absence de stimulation.

Ces dommages peuvent être causés pour de nombreuses raisons comme les traumatismes, les maladies telles que le diabète, le zona et les cancers en phase avancée, les traitements chimio-thérapeutiques ou encore une brûlure chimique. La lésion du nerf périphérique peut conduire à des états pathologiques caractérisés par la présence de douleurs spontanées continues superficielles (sensation de brûlures ou de froid douloureux) ou profondes (sensation de compression ou d'étau), de douleurs paroxystiques (décharges électriques, coup de couteau) avec à l'examen clinique une hypoesthésie ou, au contraire une hyperalgésie (réponse accrue aux stimuli nocifs), une allodynie (douleur induite par un stimulus non douloureux) ou encore une hyperpathie (douleur persistante lors de stimulations répétées non nociceptive en temps normal). Les neuropathies peuvent également être associées à des signes sensitifs tels que les paresthésies, les engourdissements, le prurit.

Les neuropathies chimio induites sont particulièrement fréquentes, invalidantes et difficiles à traiter. Elles sont doses-dépendantes. Les atteintes des nerfs périphériques représentent la majorité des atteintes neurologiques liées à la toxicité des chimiothérapies. Elles sont la conséquence d'une atteinte toxique directe de l'axone ou d'une démyélinisation et représentent le facteur limitant le plus fréquent après la toxicité hématologique.

Ainsi, devant l'apparition de neuropathies chimio-induites, les doses de chimiothérapie seront réduites voire le traitement pourra être arrêté, constituant ainsi une réelle perte de chance pour le patient.

C'est ainsi que l'on a pu constater des neuropathies à la suite de traitement par des alcaloïdes (vincristine vinblastine, vinorelbine) entrainant souvent l'atteinte de petites fibres, les dérivés du platine (oxaliplatine, cisplatine, carboplatine), les anti-topoisomérase (VP16), les inhibiteurs du protéasome (bortézomib, carfilzomib), des dérivés de thalidomide comme le lénalidomide, les taxanes tels que le taxol ou le taxotère atteignant plutôt les grosses fibres. Il existe également des neuropathies après traitement par immunothérapie comme par exemple les anti-CD20, anti-CD30, anti-CD38.

Ces douleurs chimio induites opèrent selon des mécanismes mal connus, ainsi certains auteurs pensent qu'elles sont dues à une atteinte toxique directe sur l'axone sensoriel, à une démyélinisation ou encore à une altération du métabolisme calcique liées à l'atteinte des mitochondries, site d'action du paclitaxel et de la vincristine, par exemple.

Ainsi on sait que les taxanes interviennent au niveau du ganglion rachidien, des microtubules, des mitochondries et des terminaisons nerveuses, les sels de platines interviennent au niveau de la myéline et des canaux ioniques alors que les alcaloïdes interviennent au niveau de la myéline et des microtubules.

Ces douleurs neuropathiques sont souvent réfractaires aux traitements antalgiques usuels et entraînent des diminutions de doses voire des arrêts de chimiothérapie. Elles sont aujourd'hui prises en charge par des traitements per os comprenant les antidépresseurs (Amitriptyline, Duloxetine, Venlafaxine...) et/ou les antiépileptiques (Gabapentine, Pregabaline). Malheureusement, ces traitements systémiques induisent des effets secondaires majeurs (vertiges, somnolences, pertes de mémoire, sécheresse de la bouche voire rétention d'urine, nausées...) entraînant une mauvaise observance et un contrôle des douleurs peu satisfaisant.

Ces douleurs touchent en majorité les extrémités des mains et des pieds et entraînent une altération de la qualité de vie des patients considérable avec une impotence fonctionnelle pouvant aller jusqu'à l'impossibilité de marcher, des difficultés de préhension, un sommeil altéré, l'apparition d'un syndrome dépressif voire de tendance suicidaire. Le retentissement sur la vie sociale et professionnelle peut être également très important.

L'intensité de la douleur est souvent qualifiée de sévère avec des patients qui évaluent leur douleur à plus de 7/10 sur l'Echelle Visuelle Analogique (douleur cotée de 0 à 10).

Les douleurs neuropathiques induites par chimio thérapie sont d'origine essentiellement toxique comme rappelé ci-dessus alors que les neuropathies post-zostériennes sont généralement liées à un endommagement des nerfs en raison d'une infection antérieure par le virus herpès zooster. Les nerfs endommagés ne sont plus capables de transmettre correctement les signaux de la peau vers le cerveau. Les antidépresseurs tricycliques sont des composés chimiques découverts au début des années 1950. Ils sont largement utilisés pour traiter différents troubles psychiques en particulier la dépression, les troubles de panique, les troubles obsessionnels compulsifs, l'énurésie de l'enfant, les troubles bipolaires et l'hyperactivité. Ils sont également utilisés comme antalgiques.

Ces composés sont généralement administrés par voie orale.

L'amitriptyline est un antidépresseur tricyclique découvert en 1960 qui a été fréquemment recommandé comme traitement de première intention pour la dépression majeure, le syndrome de stress post-traumatique (TSPT), le trouble anxieux généralisé (TAG), la phobie sociale (PS), le trouble de panique, la fibromyalgie, les douleurs musculo-squelettiques chroniques, l'akinésie dans la maladie de Parkinson, la cataplexie, les migraines, la maladie de Parkinson, les symptômes vasomotrices de la ménopause, l'énurésie nocturne, le trouble dysphorique prémenstruel (TDPM), le trouble bipolaire, la boulimie, les troubles obsessionnels compulsifs (TOC) et les douleurs neuropathiques.

Par le passé, les patients étaient généralement traités par l'administration d'analgésiques pour soulager la douleur. La voie orale était alors largement privilégiée.

Cependant, l'administration par voie orale de l'amitriptyline comme pour tous les antidépresseurs tricycliques présente de nombreux effets secondaires liés à leurs effets anti-cholinergiques (risque d'hypotension artérielle, de tachycardie sinusale ou supraventriculaire, rarement BAV, d'une vision floue, d'une sécheresse buccale, de flush cutanés, de rétention aiguë d'urines ou de ralentissement du transit), anti-alpha adrénergique (risque de sédation, d'hypotension d'impuissance), inhibiteurs centraux des réflexes sympathiques ou encore stabilisant de membrane (effet proarythmogène) En particulier, un des effets redoutable et craint de l'amitriptyline est l'allongement du QT pouvant entraîner le décès d'un patient qui n'aurait pas été correctement surveillé.

En particulier, lors d'une administration par voie orale de l'amitriptyline pour le traitement des douleurs neuropathiques diabétiques, il a été rapporté des cas de sédation, d'hypotension orthostatique et des effets anti-cholinergiques ((cf. notamment Kiani et al, Iran J Pharm. Res. 2015 Fall ; 14(4) :1263-8). A long terme, les patients rapportent des troubles de la mémoire, des difficultés de concentration avec un important retentissement sur la qualité de leur travail ou sur leur vie quotidienne.

Par ailleurs, l'efficacité de l'amitriptyline par voie orale est lente (il faut 5 à 7 jours de traitement pour pouvoir commencer à apprécier l'efficacité du produit), variable en fonction des patients et incomplète. Il est par conséquent souvent nécessaire d'utiliser des combinaisons d'antalgiques pour pallier ces inconvénients.

En outre, la prise orale d'antidépresseurs tricycliques a souvent mauvaise réputation auprès des patients du fait de leurs utilisations dans différents troubles psychiques.

Compte tenu des problèmes des traitements par voie orale, il a été tenté des traitements par voie topique. L'efficacité de l'amitriptyline par voie topique pour les douleurs neuropathiques n'a pas été démontrée. En particulier, l'article de Thompson et al « Systematic review of topical amitriptyline for the treatment of neuropathic pain », J. Clin. Pharm. Therm. 2015, 40, 496-503, conclut que des essais cliniques contrôlés révèlent que l'amitriptyline par voie topique n'est pas efficace dans le traitement de douleurs neuropathiques. La dose maximum utilisée est de 5% pour un malade atteint de sclérose en plaques et présentant des douleurs neuropathiques. Egalement, l'article « A phase III randomized, placebo-controlled study of topical amitriptyline and ketamine for chemotherapy-induced peripherical neuropathy », Support Care Cancer, 2014 July ; 22(7) :1807-1814, a conclu qu'une composition topique comprenant 2 % en poids de kétamine et 4 % en poids d'amitriptyline n'était pas efficace pour traiter les douleurs neuropathiques post-chimiothérapie.

Ainsi, il n'existe pas de traitement satisfaisant des douleurs neuropathiques induites par la chimiothérapie. De plus des traitements combinant la kétamine et l'amitriptyline qui semblaient donner des résultats chez des patients présentant des douleurs neuropathiques post-zostérienne ou d'origine diabétique, n'ont pas permis de remédier aux douleurs neuropathiques induites par chimiothérapie, comme relevé dans l'étude clinique en phase III précitée.

Par ailleurs les doses envisagées, malgré le caractère invalidant de ces douleurs n'ont jamais dépassé 5 % que ce soit par voie orale ou topique.

Par ailleurs, les patients souffrant de neuropathies au niveau des extrémités (pieds et mains) présentent souvent une peau endommagée voire gercée et desséchée.

L'invention a donc pour objet la mise à disposition d'une composition à base d'amitriptyline efficace en application cutanée dans le traitement de neuropathies périphériques et en particulier de neuropathies induites par chimiothérapie.

L'invention a également pour objet une composition à base d'amitriptyline permettant, en plus de pallier les douleurs neuropathiques, de retrouver une peau plus saine et hydratée.

D'autres objets de l'invention apparaîtront à la lecture de la description et des exemples qui suivent.

Il a été découvert de manière surprenante qu'une composition pharmaceutique pour une application topique comprenant dans un support pharmaceutiquement acceptable et approprié pour une application topique au moins 10 % en poids d'amitriptyline ou de l'un de sels pharmaceutiquement acceptable, administrée de manière topique, permettait de traiter efficacement les douleurs neuropathiques périphériques post-chimiothérapie (ou CIPN pour « chemotherapy-induced peripheral neuropathy »).

L'invention a donc pour objet une composition pharmaceutique comprenant dans un support pharmaceutiquement acceptable et approprié pour une application topique de 10 à 30 % en poids, par rapport au poids total de la composition, d'amitriptyline ou de l'un de ses sels pharmaceutiquement acceptable, pour son utilisation dans le traitement de douleurs neuropathiques périphériques post-chimiothérapie par voie topique.

L'application topique de la composition selon l'invention est un traitement efficace pour les douleurs neuropathiques périphériques post-chimiothérapie.

De plus, l'application topique de la composition selon l'invention présente peu, voire ne présente pas d'effets secondaires. En particulier, il n'est pas observé d'irritations cutanées à l'endroit d'application de la composition.

L'amitriptyline a la formule (I) suivante :

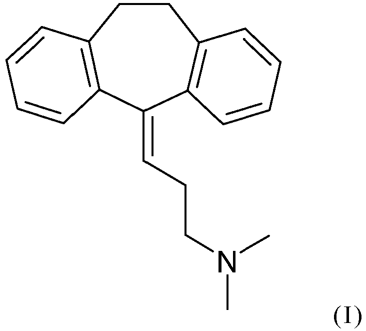

Dans le cadre de la présente invention, on entend par sel d'amitriptyline pharmaceutiquement acceptable amitriptyline, les sels compatibles avec une composition pharmaceutique c'est-à-dire destinée à être administrée à des humains. En particulier, on entend par sel d'amitriptyline pharmaceutiquement acceptable les hydrates, les solvates, les sels d'acides tel que les chlorhydrates et les clathrates de l'amitriptyline.

Comme sel tout particulièrement préféré de l'amitriptyline, on utilisera le chlorhydrate d'amitriptyline.

Comme indiqué précédemment, l'application topique de la composition selon l'invention est un traitement efficace pour les douleurs neuropathiques périphériques post-chimiothérapie.

L'application de la composition selon l'invention, dans le traitement des douleurs neuropathiques induites par chimiothérapie a permis d'obtenir des résultats particulièrement spectaculaires au regard des résultats obtenus antérieurement. C'est ainsi qu'il a été possible de réduire les douleurs classées selon l'échelle numérique simple entre 4/10 et 7/10 selon les patients, à une valeur quasi nulle pour tous les patients au bout de 1 mois de traitement.

L'utilisation de la composition selon l'invention dans le traitement des douleurs neuropathiques induites par chimiothérapie a permis de poursuivre les traitements par chimiothérapie, qui devaient souvent être interrompus ou suspendus du fait de douleurs neuropathiques sévères.

L'invention a donc également pour objet l'utilisation de la composition selon l'invention dans le cadre d'un traitement de cancers combinant chimiothérapie et traitement des douleurs neuropathiques susceptibles d'être induites par la chimiothérapie. La composition selon l'invention peut être ainsi administrée entre les cures de chimiothérapie et permettre ainsi la poursuite des traitements.

L'inventeur a par ailleurs découvert que la composition selon l'invention pouvait être appliquée à titre préventif avant un traitement de chimiothérapie et avait de façon surprenante un effet neuro protecteur, ce qui permettait d'atténuer, voire d'empêcher les douleurs neuropathiques induites par la chimiothérapie.

La composition selon l'invention peut donc également être administrée avant de débuter un traitement par chimiothérapie, l'administration de la composition selon l'invention étant poursuivie pendant et entre les cures de chimiothérapie et continuée si nécessaire après le traitement suivant l'état des douleurs neuropathiques.

La composition selon l'invention comprend de 10 à 30 % en poids, de préférence de 10 à 20% et en particulier de plus de 10% à 15% en poids d'amitriptyline ou de l'un de ses sels pharmaceutiquement acceptable par rapport au poids total de la composition.

De manière particulièrement préférée, l'amitriptyline est le seul actif pharmaceutique de la composition selon l'invention.

Dans une forme préférée la composition contient l'amitriptyline dans les proportions précitées comme seul agent traitant la douleur, en particulier sans autre agent analgésique ou agent anti dépresseur ou antiépileptique également préconisés parfois pour le traitement des douleurs neuropathiques tels que par exemple la lidocaïne, la gabapentine, la prégabaline, le baclofen, la capsaicine, la kétamine.

Ceci est particulièrement avantageux dans le cadre de l'invention puisque contrairement à l'état de l'art antérieur, l'amitriptyline seule à une teneur d'au moins 10 % en poids présente une bonne efficacité dans le traitement des douleurs neuropathiques périphériques post-chimiothérapie.

Il a été également constaté que l'application de la composition sur des explants de peau dans un modèle ex vivo conduisait à un passage systémique inférieur à 0.1% par rapport à la quantité d'amitriptyline présente dans la composition. Le passage systémique très faible permet d'éviter les effets secondaires notés pour les traitements appliqués aux neuropathies dans l'état de la technique. En particulier, la biotransformation en nortriptyline est mineure.

Les compositions pharmaceutiques selon l'invention se présentent généralement sous forme d'une émulsion huile dans eau.

Ces compositions contiennent comme composants essentiels au moins des corps gras, un ou plusieurs actifs hydratants des agents tensio-actifs non ioniques.

La phase huileuse de la composition selon l'invention comprend un ou plusieurs corps gras.

Par « corps gras », on entend, un composé organique insoluble dans l'eau à température ambiante (25°C) et à pression atmosphérique (1,013.10⁵ Pa) (solubilité inférieure à 5 % en poids, et de préférence inférieure à 1 % en poids, encore plus préférentiellement inférieure à 0,1 % en poids). Ils présentent dans leur structure au moins une chaîne hydrocarbonée comportant au moins 6 atomes de carbone et/ou un enchaînement d'au moins deux groupements siloxane. En outre, les corps gras sont généralement solubles dans des solvants organiques dans les mêmes conditions de température et de pression, comme par exemple le chloroforme, le dichlorométhane, le tétrachlorure de carbone, l'éthanol, le benzène, le toluène, le tétrahydrofurane (THF), l'huile de vaseline ou le décaméthylcyclopentasiloxane.

De préférence, le ou les corps gras sont choisis parmi les huiles synthétiques, animales, minérales ou végétales, les huiles siliconées, les acides gras, les alcools gras, les cires, les gommes et les mélanges de ces composés.

Comme exemple d'huile minérale, on peut citer les huiles de paraffine de viscosités variées.

A titre d'huile végétale, on peut notamment citer l'huile d'amande douce, l'huile de palme, l'huile de soja, l'huile de sésame et l'huile de tournesol.

A titre d'huile animale, on peut notamment citer la lanoline, le squalène, l'huile de poisson et l'huile de vison.

A titre d'huile synthétique, on peut notamment citer les esters d'alcool et d'acide gras tels que l'isononanoate de cétéaryle, le palmitate d'isopropyle et les triglycérides caprylique/caprylate.

Comme exemple d'huile de silicone, on peut notamment citer la diméthicone et la cyclométhicone.

Comme exemple d'acide gras, on peut notamment citer l'acide stéarique et l'acide palmitique.

Comme exemple d'alcool gras, on peut notamment citer l'alcool stéarylique, l'alcool cétostéarylique et l'alcool cétylique.

A titre de cire, on peut notamment citer la cire d'abeille (ou cera alba), la cire de carnauba et la cire de candelilla.

A titre de gomme, on peut notamment citer la gomme de silicone.

De manière particulièrement préférée, le ou les corps gras de la composition selon l'invention sont choisis parmi les huiles minérales, les acides gras, les cires et les mélanges de ces composés.

De manière tout particulièrement préférée, la composition selon l'invention comprend un mélange d'une ou plusieurs huiles minérales, d'un ou plusieurs acides gras et d'une ou plusieurs cires.

Le ou les corps gras représentent de préférence de 15 à 25 % en poids, par rapport au poids total de la composition et en particulier 20 à 25 % en poids, par rapport au poids total de la composition.

La composition selon l'invention peut comprendre également un ou plusieurs tensioactifs de préférence non ioniques, oxyéthylénés ou non.

De manière particulièrement préférée, la composition selon l'invention comprend un ou plusieurs tensioactifs non-ioniques non-oxyéthylénés.

Les compositions selon l'invention peuvent également contenir des systèmes auto-émulsionnant glucolipidiques, tels que des mélanges d'alcool gras et d'alkyl glycosides ayant 10 à 16 atomes de carbone et en particulier un mélange d'alcool cétylstéarylique et de cétéaryl glucoside.

Le ou les tensioactifs non-ioniques peuvent être avantageusement choisis parmi les esters de sorbitan les esters de glycérol, et les mélanges de ces composés, les polaxamères.

A titre d'ester de sorbitan, on peut notamment citer le stéarate de sorbitan ou l'oléate de sorbitan.

A titre d'ester de glycérol, on peut notamment citer le stéarate de glycérol.

De manière préférée, la composition selon l'invention comprend un mélange d'un ou plusieurs esters de sorbitan et d'un ou plusieurs esters de glycérol.

Avantageusement, le ou les tensioactifs utilisables dans la composition selon l'invention représentent, lorsqu'ils sont présents, de 2 à 8 % de préférence 2 à 5% en poids par rapport au poids total de la composition.

La composition selon l'invention peut comprendre également un ou plusieurs agents gélifiants.

Selon l'invention, un agent gélifiant est tout composé qui, ajouté à une composition, augmente la viscosité de ladite composition, l'agent gélifiant représentant de 0,01 à 4 % en poids, de préférence de 0,01 à 1 % en poids par rapport au poids total de la composition.

En augmentant la viscosité de la composition selon l'invention, celle-ci est plus stable au cours du temps.

Le ou les agents gélifiants utilisables dans la composition selon l'invention sont de préférence choisis parmi les polymères carboxyvinyliques (carbomer), les dérivés cellulosiques, les gommes de xanthane, les gommes végétales, les silicates aluminium/magnésium, les gommes de guar, les polymères polyacrylamide, les copolymères d'acrylate les amidons modifiés, et les mélanges de ces composés.

A titre de polymère carboxyvinylique (carbomer), on peut notamment citer Carbopol 981, Carbopol ETD 2020, Carbopol 980, Carbopol Ultrez 10 NF et Pemulen TR1, vendus par Lubrizol.

A titre de dérivé cellulosique, on peut notamment citer l'hydroxypropylméthylcellulose et l'hydroxyéthylcellulose.

A titre de silicate aluminium/magnésium, on peut notamment citer Veegum K et Veegum Ultra vendus par Vanderbilt.

En tant que polymère polyacrylamide, on peut notamment citer le mélange polyacrylamide/C₁₃-₁₄ isoparaffine/laureth-7, par exemple celui vendu par SEPPIC sous la marque Sepigel 305.

A titre d'amidon modifié, on peut notamment citer Structures Solanace vendu par Akzo Nobel.

De manière préférée selon l'invention, le ou les agents gélifiants utilisables selon l'invention sont choisis parmi les polymères carboxyvinyliques (carbomer).

Le ou les agents gélifiants utilisables dans la composition selon l'invention représentent, lorsqu'ils sont présents, de préférence, de 0,1 à 4 % en poids par rapport au poids total de la composition.

La composition selon l'invention comprend avantageusement de l'eau.

La composition selon l'invention comprend, dans un mode de réalisation préféré un ou plusieurs actifs hydratants.

Un actif hydratant est un actif apte à réduire l'état de sécheresse d'un épiderme.

Ainsi par « actif hydratant », on entend généralement un composé agissant sur la fonction barrière, en vue de maintenir l'hydratation du stratum cornéum, ou un composé occlusif.

On peut citer notamment les céramides, les composés à base sphingoides, les lécithines, les glycosphingolipides, les phospholipides, le cholestérol et ses dérivés, les phytostérols (stigmastérol, β-sitostérol, campestérol), le 1 -2 diacylglycérol, la 4-chromanone, les triterpènes pentacycliques, des glycosaminoglycanes, des sucres, polysaccharide, l'urée et la glycérine.

De manière préférée, l'actif hydratant est la glycérine.

Avantageusement, le ou les actifs hydratants utilisables dans la composition selon l'invention représentent, lorsqu'ils sont présents, de 7 à 15 % en poids par rapport au poids total de la composition.

La composition selon l'invention peut comprendre également un ou plusieurs additifs ou combinaisons d'additifs choisis parmi les conservateurs, les stabilisants, les exhausteurs de goût et les ajusteurs de pH.

A titre de conservateur, on peut notamment citer le phénoxyéthanol.

Bien entendu, l'homme du métier choisira les différents additifs ou combinaisons d'additifs en prenant bien soin que les propriétés intrinsèquement associées à la composition selon l'invention, ne soient pas ou soient faiblement altérées par les ajouts envisagés.

Les additifs, lorsqu'ils sont présents dans la composition selon l'invention, représentent généralement chacun de 0,001 à 20 % en poids par rapport au poids total de la composition.

Dans un mode de réalisation préférée de l'invention, la composition comprend :
- de 10 à 30 %, de préférence de 10 à 20 % en poids, plus préférentiellement de plus de 10 à 15 % en poids d'amitriptyline ou de l'un de ses sels pharmaceutiquement acceptable,
- de 2 à 8 % en poids d'un ou plusieurs tensioactifs non ioniques,
- de 15 à 25 % en poids d'un ou plusieurs corps gras,
- de 0,1 à 4 % en poids d'un ou plusieurs agents gélifiants,
- de 7 à 15 % en poids d'un ou plusieurs actifs hydratants,
- optionnellement de 0 à 3 % en poids d'un ou plusieurs conservateurs,
- optionnellement de 0 à 1 % en poids d'un ou plusieurs ajusteurs de pH, de façon à maintenir le pH aux environs de 7, en particulier entre 6,5 et 7,5.
- de l'eau.

Ces compositions sont particulièrement efficaces dans le traitement de douleurs neuropathiques induites par chimiothérapie, car elles permettent de traiter non seulement la douleur de manière efficace mais également de restaurer la peau souvent déshydratée au niveau des extrémités douloureuses.

De manière préférée, le ou les tensioactifs, le ou les corps gras, le ou les agents gélifiants, le ou les actifs hydratants, et le ou les conservateurs sont tels que définis ci-avant.

De manière particulièrement préférée dans ce mode de réalisation, la composition selon l'invention comprend :
- de 10 à 30 %, de préférence de 10 à 20 % en poids, plus préférentiellement de 10,5 à 15 % en poids d'amitriptyline ou de l'un de ses sels pharmaceutiquement acceptable,
- de 2 à 8 % en poids d'un ou plusieurs tensioactifs choisis parmi les esters de sorbitan, les esters de glycérol, et les mélanges de ces composés, ou autres tensio-actifs permettant la stabilisation de la formule :
- de 15 à 25 % en poids d'un ou plusieurs corps gras dont les huiles minérales, les acides gras, les cires et les mélanges de ces composés,
- de 0,1 à 4 % en poids d'un ou plusieurs agents gélifiants dont les polymères carboxyvinyliques,
- de 7 à 15 % en poids d'un ou plusieurs actifs hydratants,
- optionnellement de 0 à 3 % en poids d'un ou plusieurs conservateurs,
- optionnellement de 0 à 1 % en poids d'un ou plusieurs ajusteurs de pH,
- de l'eau.

De manière tout particulièrement préférée dans ce mode de réalisation, la composition selon l'invention comprend :
- de 10 à 30 %, de préférence de 10 à 20 % en poids, plus préférentiellement de 10,5 à 15 % en poids d'amitriptyline ou de l'un de ses sels pharmaceutiquement acceptable,
- de 2 à 8 % en poids d'un mélange plusieurs tensio-actifs non ioniques dont un ou plusieurs esters de sorbitan et d'un ou plusieurs esters de glycérol,
- de 15 à 25 % en poids d'un mélange d'une ou plusieurs huiles minérales, d'un ou plusieurs acides gras et d'une ou plusieurs cires,
- de 0,1 à 4 % en poids d'un ou plusieurs polymères carboxyvinyles (carbomer),
- de 7 à 15 % en poids de glycérine,
- optionnellement de 0 à 3 % en poids d'un ou plusieurs conservateurs,
- optionnellement de 0 à 1 % en poids d'un ou plusieurs ajusteurs de pH,
- de l'eau.

En particulier, ce mode de réalisation permet de diminuer, voire de supprimer les effets secondaires associé à l'absorption d'amitriptyline, en particulier les irritations cutanées à l'endroit d'application de la composition.

Ce mode de réalisation permet également d'obtenir une bonne stabilité dans le temps de la composition selon l'invention à température ambiante mais également à des températures de stockage plus élevées (45°C par exemple).

Enfin, ce mode de réalisation permet de manière avantageuse de faciliter la pénétration de l'amitriptyline à travers la peau sans passage systémique. La majorité de l'amitriptyline est concentrée dans le derme. On obtient ainsi une bonne efficacité thérapeutique avec une bonne tolérance.

Le pH des compositions selon l'invention est de préférence compris entre 5 et 8 et est ajusté par une base de type NaOH ou triéthanolamine

La composition selon l'invention est une composition topique.

La composition selon l'invention peut se présenter sous forme liquide, pâteuse, ou solide, et plus particulièrement sous forme d'onguents, de crèmes, de laits, de pommades. De manière préférée, la composition selon l'invention est sous forme de crème légère et onctueuse.

Les exemples suivants illustrent la composition selon l'invention et les avantages de cette composition. Cependant, ils ne représentent en rien une limitation de la présente invention mais illustrent simplement l'invention.

### Exemples

### Exemple 1 :

### Composition sous forme d'une émulsion huile dans eau:

| | |
|---|---|
| Amitriptyline | 10mg |
| Stéarate de glycérol | 1 mg |
| Autres tensio-actifs | 1 mg |
| Paraffinum liquidum | 12 mg |
| Acide palmitique | 1 mg |
| Acide stéarique | 1 mg |
| Cire d'abeille (cera alba) | 1 mg |
| Carbomer | 0,1 mg |
| Glycérine | 10 mg |
| Agent d'ajustement du pH qs | pH 6,9 |
| Conservateur | qs |
| Eau | qs 100 mg |

La crème ainsi obtenue a été appliquée une fois matin et soir sur les zones douloureuses d'une population de 31 patients présentant des douleurs neuropathiques périphériques post chimiothérapie. Elle a été appliquée sur les mains et les pieds.

Parmi les patients pris en charge, il a été distingué 3 groupes suivant l'ancienneté des douleurs neuropathiques.

Le groupe GR1 (11 patients) a été pris en charge dans un délai d'un mois après l'apparition des douleurs neuropathiques. Ces patients ressentent des douleurs neuropathiques d'intensité modérée (Echelle Visuelle Analogique, EVA= 4/10) de type décharges électriques, brulures, fourmillement. Du fait de l'apparition de ces douleurs invalidantes, les doses de chimiothérapie ont dues être diminuées dans la majorité des cas, la chimiothérapie a dû être arrêtée pour 2 patients. L'application de la crème d'amitriptyline ci-dessus, pendant 3 à 5 jours est efficace à 100% et réduit totalement la douleur (EVA 0/10). Le traitement est arrêté, sans récidive, au bout de 1 mois permettant de reprendre la chimiothérapie ou de ré-augmenter les doses.

Le groupe GR2, les patients (13) ressentent des douleurs neuropathiques des extrémités d'intensité modérée à sévère (EVA compris entre 5 et 7/10), de type brûlures, décharges électriques, fourmillement, sensation « d'œdème » des zones atteintes. L'application de la crème amitriptyline à 10% est efficace (EVA 2-3/10) au bout de 15 jours de traitement. A un mois, les douleurs ont disparu (EVA 0/10) chez la totalité des patients. Les patients traités pour des neuropathies post-chimiothérapie poursuivent le traitement pendant la durée des cures de chimiothérapie à titre préventif.

Le groupe GR3, les patients (7) ressentent des douleurs neuropathiques d'intensité sévère (EVA supérieure à 7/10) à types de brûlures, décharges électriques, coup de poignard, fourmillements, sensation « d'œdème » au niveau des zones atteintes. Le retentissement fonctionnel est majeur (difficulté à la préhension lorsque les mains sont touchées, difficulté à la marche lorsque les pieds sont touchés, impossibilité à mettre des chaussures fermées, difficulté à mettre des vêtements sur la zone atteinte) et les patients présentent un syndrome dépressif associé aux douleurs neuropathiques. Pour ce dernier groupe de patient, la crème amitriptyline 10% commence à être efficace (perte de 3 points d'EVA) à partir d'un mois de traitement. Le traitement est poursuivi pendant 3 mois. (EVA inférieure à 2/10).

### Exemple 2

### Composition sous forme d'une émulsion huile dans eau :

| | |
|---|---|
| Amitriptyline | 15mg |
| Stéarate de glycérol | 1mg |
| Autres tensio-actifs | 1 mg |
| Paraffinum liquidum | 12 mg |
| Acide palmitique | 1 mg |
| Acide stéarique | 1 mg |
| Cire d'abeille (cera alba) | 1 mg |
| Carbomer | 0,1 mg |
| Glycérine | 10 mg |
| Agent d'ajustement du pH qs | pH 6,9 |
| Conservateur | qs |
| Eau | qs 100 mg |

La composition sous forme de crème a été administrée à des patients ressentent des douleurs neuropathiques d'intensité sévère (EVA supérieure à 7/10) à types de brûlures, décharges électriques, coup de poignard, fourmillements, sensation « d'œdème » au niveau des zones atteintes. L'administration de la crème apporte un très bon contrôle des douleurs en 1 mois de traitement (EVA comprise en 0 et 2/10).

### Exemple 3 :

La crème de l'exemple 1 a été appliquée une fois matin et soir sur les zones douloureuses d'une population de 5 patients présentant des douleurs neuropathiques périphériques post zostériennes.

Les patients ressentent des douleurs neuropathiques des extrémités d'intensité modérée à sévère (EVA compris entre 6 et 8/10), de type brûlures, décharges électriques, fourmillement, sensation « d'œdème » des zones atteintes. L'application de la crème de l'exemple 1 sur le thorax (4) et sur les cuisses (1) est efficace (EVA 2-3/10) au bout de 15 jours de traitement. A un mois, les douleurs ont disparu (EVA 0/10) chez la totalité des patients. Les patients traités peuvent arrêter le traitement.

### Exemple 4 :

On a préparé la crème suivante sous forme d'émulsion huile dans eau :

| | |
|---|---|
| Amitriptyline | 10 mg |
| Stéarate de glycérol | 2 mg |
| Stéarate de sorbitan | 1 mg |
| Paraffinum liquidum | 8 mg |
| Cétéaryléthylhexanoate | 5 mg |
| Acide palmitique | 1 mg |
| Acide stéarique | 1 mg |
| Cire d'abeille (cera alba) | 2 mg |
| Carbomer | 0,2 mg |
| Copolymère hydroxyéthyl acrylate/sodium acryloyldiméthyl taurate | 0,1 mg |
| Glycérine | 10 mg |
| Agent d'ajustement du pH qs | pH 6,9 |
| Conservateur | qs |
| Eau | qs 100 mg |

La crème a été appliquée une fois matin et soir sur les zones douloureuses d'un patient présentant des douleurs neuropathiques périphériques post-diabétiques.

Le patient ressent des douleurs neuropathiques d'intensité sévère (EVA supérieure à 7/10) à types de brûlures, décharges électriques, coup de poignard, fourmillements, sensation « d'œdème » au niveau des zones atteintes. Le retentissement fonctionnel est majeur (difficulté à la préhension lorsque les mains sont touchées, difficulté à la marche lorsque les pieds sont touchés, impossibilité à mettre des chaussures fermées, difficulté à mettre des vêtements sur la zone atteinte). La crème amitriptyline 10% commence à être efficace (perte de 3 points d'EVA) à partir d'un mois de traitement. Le traitement est poursuivi pendant 3 mois. (EVA inférieure à 2/10).

### Exemple 5 :

2 patients atteints de cancer du côlon et ayant comme chimiothérapie des cures de R-CHOP, ayant fait après la 1ère cure une neuropathie sévère au niveau des mains à type de fourmillements, sensation de froid douloureux très intense EVA 7-8/10 ont été traités. Devant l'intensité de la neuropathie, la 2eme cure de R-CHOP avait été repoussée.

7 jours avant la 2eme cure, les patients ont appliqués sur les mains la crème amitriptyline 10% et ont poursuivi l'application 7 jours après la cure de chimiothérapie sans souffrir de neuropathie. (EVA 1-2/10 sans sensation de froid douloureux.)

### Exemple 6 :

Etude *ex vivo* de l'absorption percutanée de l'amitriptyline et de la présence du métabolite pharmacologiquement actif : nortriptyline.

On a appliqué une composition sous forme de crème contenant 10% en poids de chlorhydrate d'amitriptyline sur des échantillons de peau humaine. L'expérience a été répétée 3 fois avec 3 échantillons de peau de 3 donneurs différents, soit 9 échantillons. Les échantillons de peau sont montés dans une cellule de Frantz et sont portées à une température de surface de 32°C±1°C.

La formulation contenant 10% en poids de chlorhydrate d'amitriptyline en l'étendant de façon homogène à l'aide d'une spatule sur chaque échantillon de peau à raison de 10 mg par cellule, correspondant à 5mg /cm² de peau.

On rince les échantillons de peau 16 heures après application.

Chaque échantillon de peau a été placé avec une pince à épiler sur un papier absorbant (derme vers le bas).

On enlève le stratum corneum en utilisant des bandes adhésives.

Après élimination du stratum corneum l'échantillon est perforé. L'épiderme est ensuite séparé du derme. Chacun d'eux est placé dans des fioles séparées.

On a procédé ensuite à l'extraction des différents échantillons.

Ces essais ont permis de constater que 90,6 à 98% d'amitriptyline reste à la surface de la peau. Environ 74% de l'amitriptyline est présente dans le derme contre 26% dans l'épiderme.

La biodisponibilité de l'amitriptyline est de 22, 5 µg.

On a observé un passage systémique inférieur à 0,1%, et une très faible biotransformation de l'amitriptyline en nortriptyline de l'ordre de 25ng.

## Revendications

1. Composition pharmaceutique comprenant dans un support pharmaceutiquement acceptable et approprié pour une application topique de 10 à 30 % en poids, par rapport au poids total de la composition, d'amitriptyline ou de l'un de ses sels pharmaceutiquement acceptables, pour son utilisation dans le traitement de douleurs neuropathiques périphériques post-chimiothérapie par voie topique.

2. Composition pour son utilisation selon la revendication 1 pour son utilisation dans le traitement de douleurs neuropathiques périphériques post-chimiothérapie, par application sur les parties périphériques (mains et pieds)

3. Composition pour son utilisation selon la revendication 1 ou 2 **caractérisée en ce qu'**elle comprend de 10 à 20 % en poids, de préférence plus de 10 à 15 % en poids, par rapport au poids total de la composition, d'amitriptyline ou de l'un de ses sels pharmaceutiquement acceptable.

4. Composition pour son utilisation selon l'une quelconque des revendications 1 à 3 contenant l'amitriptyline comme seul agent de traitement des douleurs neuropathiques.

5. Composition pour son utilisation selon l'une quelconque des revendications 1 à 4 pour son utilisation à titre préventif avant un traitement de chimiothérapie en vue d'atténuer, voire d'empêcher les douleurs neuropathiques périphériques induites par la chimiothérapie.

6. Composition pour son utilisation selon l'une quelconque des revendications 1 à 4 pour son utilisation dans le traitement de cancers comportant des séances de chimiothérapie, la composition étant administrée entre les séances de chimiothérapie pour remédier ou prévenir des douleurs neuropathiques susceptibles d'être induites par la chimiothérapie.

7. Composition pour son utilisation selon l'une quelconque des revendications 1 à 6 pour son utilisation dans le traitement de cancers comportant des séances de chimiothérapie, la composition étant administrée à titre préventif avant la séance de chimiothérapie, puis pendant et entre les cures de chimiothérapie et continuée si nécessaire après le traitement par chimiothérapie suivant l'état des douleurs neuropathiques.

8. Composition pour son utilisation selon l'une quelconque des revendications 1 à 7 se présentant sous forme d'une émulsion huile dans eau et comprenant au moins des corps gras, un ou plusieurs actifs hydratants et des agents tensioactifs non ioniques.

9. Composition pour son utilisation selon l'une quelconque des revendications précédentes, **caractérisée en ce qu'**elle comprend un ou plusieurs corps gras, de préférence choisis parmi les huiles synthétiques, animales, minérales ou végétales, les huiles siliconées, les acides gras, les alcools gras, les cires, les gommes et les mélanges de ces composés, plus préférentiellement choisis parmi les huiles minérales, les acides gras, les cires et les mélanges de ces composés, et en particulier la composition comprend un mélange d'une ou plusieurs huiles minérales, d'un ou plusieurs acides gras et d'une ou plusieurs cires.

10. Composition pour son utilisation selon l'une quelconque des revendications précédentes, **caractérisée en ce qu'**elle comprend un ou plusieurs tensioactifs non-ioniques choisi parmi les esters de sorbitan, les esters de glycérol, et les mélanges de ces composés.

11. Composition pour son utilisation selon l'une quelconque des revendications précédentes, **caractérisée en ce qu'**elle comprend un ou plusieurs agents gélifiants, de préférence choisis parmi les polymères carboxyvinyliques, les dérivés cellulosiques, les gommes de xanthane, les gommes végétales, les silicates aluminium/magnésium, les gommes de guar, les polymères polyacrylamide, les copolymères d'acrylate les amidons modifiés, et les mélanges de ces composés, et plus préférentiellement choisis parmi les polymères carboxyvinyles.

12. Composition pour son utilisation selon l'une quelconque des revendications précédentes, **caractérisée** en qu'elle comprend :
- de 10 à 30 %, de préférence de 10 à 20 % en poids, plus préférentiellement plus de 10 à 15 % en poids d'amitriptyline ou de l'un de ses sels pharmaceutiquement acceptable,
- de 2 à 8 % en poids d'un ou plusieurs tensioactifs,
- de 15 à 25 % en poids d'un ou plusieurs corps gras,
- de 0,1 à 4 % en poids d'un ou plusieurs agents gélifiants,
- de 7 à 15 % en poids d'un ou plusieurs actifs hydratants,
- optionnellement de 0 à 3 % en poids d'un ou plusieurs conservateurs,
- optionnellement de 0 à 1 % en poids d'un ou plusieurs ajusteurs de pH,
- de l'eau.

13. Composition pour son utilisation selon la revendication 12, **caractérisée en ce qu'**elle comprend :
- de 10 à 30 %, de préférence de 10 à 20 % en poids, plus préférentiellement de 10,5 à 15 % en poids d'amitriptyline ou de l'un de ses sels pharmaceutiquement acceptable,
- de 2 à 8 % en poids d'un ou plusieurs tensioactifs choisis parmi les esters de sorbitan, les esters de glycérol, et les mélanges de ces composés,
- de 15 à 25 % en poids d'un ou plusieurs corps gras choisis parmi les huiles minérales, les acides gras, les cires et les mélanges de ces composés,
- de 0,1 à 4 % en poids d'un ou plusieurs agents gélifiants choisis parmi les polymères carboxyvinyles,
- de 7 à 15 % en poids d'un ou plusieurs actifs hydratants,
- optionnellement de 0 à 3 % en poids d'un ou plusieurs conservateurs,
- optionnellement de 0 à 1 % en poids d'un ou plusieurs ajusteurs de pH,
- de l'eau.

14. Composition pour son utilisation selon la revendication 12 ou 13, **caractérisée en ce qu'**elle comprend :
- de 10 à 30 %, de préférence de 10 à 20 % en poids, plus préférentiellement de 10,5 à 15 % en poids d'amitriptyline ou de l'un de ses sels pharmaceutiquement acceptable,
- de 2 à 8 % en poids d'un mélange d'un ou plusieurs esters de sorbitan et d'un ou plusieurs esters de glycérol,
- de 15 à 25 % en poids d'un mélange d'une ou plusieurs huiles minérales, d'un ou plusieurs acides gras et d'une ou plusieurs cires,
- de 0,1 à 4 % en poids d'un ou plusieurs polymères carboxyvinyles,
- de 7 à 15 % en poids de glycérine,
- optionnellement de 0 à 3 % en poids d'un ou plusieurs conservateurs,
- optionnellement de 0 à 1 % en poids d'un ou plusieurs ajusteurs de pH,
- de l'eau.

15. Composition pour son utilisation selon l'une quelconque des revendications précédentes, **caractérisée** en qu'elle est sous forme de crème.

## Patentansprüche

1. Pharmazeutische Zusammensetzung, die in einem pharmazeutisch unbedenklichen Träger, welcher sich weiterhin für eine topische Anwendung eignet, 10 bis 30 Gewichts-% an Amitriptylin oder an einem von dessen pharmazeutisch unbedenklichen Salzen, unter Bezugnahme auf das Gesamtgewicht der Zusammensetzung umfasst, zur Verwendung derselben in der Behandlung postchemotherapeutischer peripherer neuropathischer Schmerzen auf topischem Wege.

2. Zusammensetzung zur Verwendung derselben nach Anspruch 1, wobei sie in der Behandlung postchemotherapeutischer peripherer neuropathischer Schmerzen verwendet wird, indem sie auf die peripheren Bereiche (Hände und Füße) aufgebracht wird.

3. Zusammensetzung zur Verwendung derselben nach Anspruch 1 oder 2, **dadurch gekennzeichnet, dass** sie 10 bis 20 Gewichts-%, vorzugsweise mehr als 10 bis 15 Gewichts-%, unter Bezugnahme auf das Gesamtgewicht der Zusammensetzung, an Amitriptylin oder an einem von dessen pharmazeutisch unbedenklichen Salzen umfasst.

4. Zusammensetzung zur Verwendung derselben nach einem beliebigen der Ansprüche 1 bis 3, wobei sie Amitriptylin als einziges Mittel zur Behandlung neuropathischer Schmerzen enthält.

5. Zusammensetzung zur Verwendung derselben nach einem beliebigen der Ansprüche 1 bis 4, wobei diese im Vorfeld einer chemotherapeutischen Behandlung vorbeugend verwendet wird, um die peripheren neuropathischen Schmerzen, wie sie von der Chemotherapie bewirkt werden, abzuschwächen oder sogar zu verhindern.

6. Zusammensetzung zur Verwendung derselben nach einem beliebigen der Ansprüche 1 bis 4, wobei sie in Krebsbehandlungen verwendet wird, welche Chemotherapiesitzungen aufweisen, wobei die Zusammensetzung zwischen den Chemotherapiesitzungen verabreicht wird, um neuropathische Schmerzen zu beheben oder zu verhüten, welche von der Chemotherapie bewirkt werden könnten.

7. Zusammensetzung zur Verwendung derselben nach einem beliebigen der Ansprüche 1 bis 6, wobei sie in Krebsbehandlungen verwendet wird, welche Chemotherapiesitzungen aufweisen, wobei die Zusammensetzung vorbeugend im Vorfeld der Chemotherapiesitzung und anschließend während der chemotherapeutischen Kuren und zwischen denselben verabreicht wird, wobei dies erforderlichenfalls nach der Behandlung mittels Chemotherapie fortgesetzt wird, in Abhängigkeit vom Zustand der neuropathischen Schmerzen.

8. Zusammensetzung zur Verwendung derselben nach einem beliebigen der Ansprüche 1 bis 7, wobei sie in Form einer Öl-in-Wasser-Emulsion vorliegt und mindestens einen Fettstoff, einen oder mehrere feuchtigkeitsspendende Wirkstoffe sowie nichtionische Tenside umfasst.

9. Zusammensetzung zur Verwendung derselben nach einem beliebigen der vorhergehenden Ansprüche, **dadurch gekennzeichnet, dass** sie einen oder mehrere Fettstoffe umfasst, die vorzugsweise aus den synthetischen, tierischen, mineralischen oder pflanzlichen Ölen, den Silikonölen, den Fettsäuren, den Fettalkoholen, den Wachsen, den Gummen und den Mischungen dieser Verbindungen ausgewählt ist, wobei sie stärker bevorzugt aus den mineralischen Ölen, den Fettsäuren, den Wachsen und den Mischungen dieser Verbindungen ausgewählt ist und wobei die Zusammensetzung insbesondere eine Mischung aus einem oder mehreren mineralischen Ölen, aus einer oder mehreren Fettsäuren und einem oder mehreren Wachsen umfasst.

10. Zusammensetzung zur Verwendung derselben nach einem beliebigen der vorhergehenden Ansprüche, **dadurch gekennzeichnet, dass** sie ein oder mehrere nichtionische Tenside umfasst, welche aus den Sorbitanestern, den Glycerinestern und den Mischungen dieser Verbindungen ausgewählt sind.

11. Zusammensetzung zur Verwendung derselben nach einem beliebigen der vorhergehenden Ansprüche, **dadurch gekennzeichnet, dass** sie einen oder mehrere gelbildende Stoffe umfasst, die vorzugsweise aus den Carboxyvinylpolymeren, den Cellulosederivaten, den Xanthanen, den pflanzlichen Gummen, den Aluminium-/Magnesiumsilikaten, den Guarkernmehlen, den Polyacrylamidpolymeren, den Acrylatcopolymeren, den modifizierten Stärken und den Mischungen dieser Verbindungen ausgewählt sind, wobei sie stärker bevorzugt aus den Carboxyvinylpolymeren ausgewählt sind.

12. Zusammensetzung zur Verwendung derselben nach einem beliebigen der vorhergehenden Ansprüche, **dadurch gekennzeichnet, dass** sie Folgendes umfasst:
- 10 bis 30 %, vorzugsweise 10 bis 20 % nach Gewicht, stärker bevorzugt 10 bis 15 Gewichts-% an Amitriptylin oder an einem von dessen pharmazeutisch unbedenklichen Salzen,
- 2 bis 8 Gewichts-% an einem oder mehreren Tensiden,
- 15 bis 25 Gewichts-% an einem oder mehreren Fettstoffen,
- 0,1 bis 4 Gewichts-% an einem oder mehreren gelbildenden Mitteln,
- 7 bis 15 Gewichts-% an einem oder mehreren feuchtigkeitsspendenden Wirkstoffen,
- möglicherweise 0 bis 3 Gewichts-% an einem oder mehreren Konservierungsstoffen,
- möglicherweise 0 bis 1 Gewichts-% an einem oder mehreren pH-Regulatoren,
- Wasser.

13. Zusammensetzung zur Verwendung derselben nach Anspruch 12, **dadurch gekennzeichnet, dass** sie Folgendes umfasst:
- 10 bis 30 %, vorzugsweise 10 bis 20 % nach Gewicht, stärker bevorzugt 10,5 bis 15 Gewichts-% an Amitriptylin oder an einem von dessen pharmazeutisch unbedenklichen Salzen,
- 2 bis 8 Gewichts-% eines oder mehrerer Tenside, die aus den Sorbitanestern, den Glycerinestern und den Mischungen dieser Verbindungen ausgewählt sind,
- 15 bis 25 Gewichts-% eines oder mehrerer Fettstoffe, die aus den mineralischen Ölen, den Fettsäuren, den Wachsen und den Mischungen dieser Verbindungen ausgewählt sind,
- 0,1 bis 4 Gewichts-% an einem oder mehreren gelbildenden Mitteln, die aus den Carboxyvinylpolymeren ausgewählt sind,
- 7 bis 15 Gewichts-% an einem oder mehreren feuchtigkeitsspendenden Wirkstoffen,
- möglicherweise 0 bis 3 Gewichts-% an einem oder mehreren Konservierungsstoffen,
- möglicherweise 0 bis 1 Gewichts-% an einem oder mehreren pH-Regulatoren,
- Wasser.

14. Zusammensetzung zur Verwendung derselben nach Anspruch 12 oder 13, **dadurch gekennzeichnet, dass** sie Folgendes umfasst:
- 10 bis 30 %, vorzugsweise 10 bis 20 % nach Gewicht, stärker bevorzugt 10,5 bis 15 Gewichts-% an Amitriptylin oder an einem von dessen pharmazeutisch unbedenklichen Salzen,
- 2 bis 8 Gewichts-% einer Mischung aus einem oder mehreren Sorbitanestern und aus einem oder mehreren Glycerinestern,
- 15 bis 25 Gewichts-% einer Mischung aus einem oder mehreren Mineralölen, aus einer oder mehreren Fettsäuren und aus einem oder mehreren Wachsen,
- 0,1 bis 4 Gewichts-% an einem oder mehreren Carboxyvinylpolymeren,
- 7 bis 15 Gewichts-% an Glycerin,
- möglicherweise 0 bis 3 Gewichts-% an einem oder mehreren Konservierungsstoffen,
- möglicherweise 0 bis 1 Gewichts-% an einem oder mehreren pH-Regulatoren,
- Wasser.

15. Zusammensetzung zur Verwendung derselben nach einem beliebigen der vorhergehenden Ansprüche, **dadurch gekennzeichnet, dass** sie als Creme vorliegt.

## Claims

1. Pharmaceutical composition comprising, in a pharmaceutically acceptable support suitable for topical application, from 10% to 30% by weight, relative to the total weight of the composition, of amitriptyline or of a pharmaceutically acceptable salt thereof, for use in the topical treatment of chemotherapy-induced peripheral neuropathic pain.

2. Composition for use according to Claim 1, for use in the treatment of chemotherapy-induced peripheral neuropathic pain, by application to the peripheral parts (hands and feet).

3. Composition for use according to Claim 1 or 2, **characterized in that** it comprises from 10% to 20% by weight, preferably more than 10% to 15% by weight, relative to the total weight of the composition, of amitriptyline or of a pharmaceutically acceptable salt thereof.

4. Composition for use according to any one of Claims 1 to 3, containing the amitriptyline as sole agent for treating neuropathic pain.

5. Composition for use according to any one of Claims 1 to 4, for preventive use before a chemotherapy treatment for the purpose of reducing, or even preventing, chemotherapy-induced peripheral neuropathic pain.

6. Composition for use according to any one of Claims 1 to 4, for use in cancer treatment comprising chemotherapy sessions, the composition being administered between the chemotherapy sessions in order to remedy or prevent neuropathic pain that may be chemotherapy induced.

7. Composition for use according to any one of Claims 1 to 6, for use in cancer treatment comprising chemotherapy sessions, the composition being administered preventively before the chemotherapy session, then during and between the chemotherapy regimens and continuing if necessary after the chemotherapy treatment depending on the state of the neuropathic pain.

8. Composition for use according to any one of Claims 1 to 7, in the form of an oil-in-water emulsion and comprising at least fatty substances, one or more hydrating active agents and non-ionic surfactants.

9. Composition for use according to any one of the preceding claims, **characterized in that** it comprises one or more fatty substances, preferably chosen from synthetic, animal, mineral or vegetable oils, silicone oils, fatty acids, fatty alcohols, waxes, gums and mixtures of these compounds, more preferentially chosen from mineral oils, fatty acids, waxes and mixtures of these compounds, and in particular the composition comprises a mixture of one or more mineral oils, of one or more fatty acids and of one or more waxes.

10. Composition for use according to any one of the preceding claims, **characterized in that** it comprises one or more non-ionic surfactants chosen from sorbitan esters, glycerol esters, and mixtures of these compounds.

11. Composition for use according to any one of the preceding claims, **characterized in that** it comprises one or more gelling agents, preferably chosen from carboxyvinyl polymers, cellulose-based derivatives, xanthan gums, vegetable gums, aluminium/magnesium silicates, guar gums, polyacrylamide polymers, acrylate copolymers, modified starches, and mixtures of these compounds, and more preferentially chosen from carboxyvinyl polymers.

12. Composition for use according to any one of the preceding claims, **characterized in that** it comprises:
- from 10% to 30%, preferably from 10% to 20% by weight, more preferentially more than 10% to 15% by weight of amitriptyline or of a pharmaceutically acceptable salt thereof,
- from 2% to 8% by weight of one or more surfactants,
- from 15% to 25% by weight of one or more fatty substances,
- from 0.1% to 4% by weight of one or more gelling agents,
- from 7% to 15% by weight of one or more hydrating active agents,
- optionally from 0 to 3% by weight of one or more preservatives,
- optionally from 0 to 1% by weight of one or more pH adjusters,
- water.

13. Composition for use according to Claim 12, **characterized in that** it comprises:
- from 10% to 30%, preferably from 10% to 20% by weight, more preferentially from 10.5% to 15% by weight of amitriptyline or of a pharmaceutically acceptable salt thereof,
- from 2% to 8% by weight of one or more surfactants chosen from sorbitan esters, glycerol esters, and mixtures of these compounds,
- from 15% to 25% by weight of one or more fatty substances chosen from mineral oils, fatty acids, waxes and mixtures of these compounds,
- from 0.1% to 4% by weight of one or more gelling agents chosen from carboxyvinyl polymers,
- from 7% to 15% by weight of one or more hydrating active agents,
- optionally from 0 to 3% by weight of one or more preservatives,
- optionally from 0 to 1% by weight of one or more pH adjusters,
- water.

14. Composition for use according to Claim 12 or 13, **characterized in that** it comprises:
- from 10% to 30%, preferably from 10% to 20% by weight, more preferentially from 10.5% to 15% by weight of amitriptyline or of a pharmaceutically acceptable salt thereof,
- from 2% to 8% by weight of a mixture of one or more sorbitan esters and of one or more glycerol esters,
- from 15% to 25% by weight of a mixture of one or more mineral oils, of one or more fatty acids and of one or more waxes,
- from 0.1% to 4% by weight of one or more carboxyvinyl polymers,
- from 7% to 15% by weight of glycerol,
- optionally from 0 to 3% by weight of one or more preservatives,
- optionally from 0 to 1% by weight of one or more pH adjusters,
- water.

15. Composition for use according to any one of the preceding claims, **characterized in that** it is in the form of a cream.
